# EUROPEAN PATENT APPLICATION

(11) **EP 2 439 282 A1**
(43) Date of publication of application: **11.04.2012**
(21) Application number: 10306090.1
(22) Date of filing: 06.10.2010
(51) Int. Cl.: C12Q 1/68

(54) **Method for determining a biological pathway activity**

(71) Applicant: bioMérieux, 69280 Marcy L'Etoile (FR); Hospices Civils De Lyon, 69002 Lyon (FR)
(72) Inventor: Miossec, Pierre, 69500 BRON (FR); Mougin, Bruno, 69003 LYON (FR); Paye, Malick, 69003 LYON (FR); Reynier, Frédéric, 69100 VILLEURBANNE (FR)
(74) Representative: Maureau, Philippe

(57) **Abstract**

Method for determining a level of activity of a biological pathway from a biological sample of a patient suffering from a pathology, said method comprising the steps of:
measuring the level of expression of at least three genes from a group of individuals, control individuals or patients, for which the biological pathway is inactive, said at least three genes being associated to said biological pathway, for establishing a negative reference, and
measuring level of expression of said at least three genes from a group of individuals, control individuals or patients, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein
if the correlation score is lower to the predetermined threshold that is indicative of the non activity of the biological pathway, and
if the correlation score is upper or equal to the predetermined threshold that is indicative of the activity of the biological pathway.

## Description

The present invention pertains to a method for determining a biological pathway activity, said biological pathway being associated with a disease. More precisely, the method consists in evaluating the level of activity of said pathway in a patient suffering from said disease, based on gene expression profiling. The invention also concerns the application of such method to targeted therapies.

A wide range of methods for microarray data analysis have evolved, ranging from simple fold-change approaches to many complex and computationally demanding techniques. Gene expression profiling by microarray technology has become a widely used strategy for investigating the molecular mechanisms underlying many complex diseases. However, the analysis is further complicated by the biological heterogeneity encountered in most of the diseases.

A common observation in the analysis of gene expression is that many genes show similar expression patterns (1) which may share biological functions under common regulatory control. Moreover, these co-expressed genes are frequently clustered according to their expression patterns in subsets of experimental conditions (2). Thus, gene co-expression instead of differential expression could be informative as well.

However, the method commonly used in the literature does not take into account the activation status of the biological signature, which can generate some misclassification (3-7).

Thus, the inventors have developed a method for determining a level of activity of a biological pathway of a patient suffering from a disease, providing a response indicative of the activity or non activity of said biological pathway, which avoids misclassifications.

The method of the present invention permits to identify truly active biological networks associating only with high levels of correlation of biological signature components. Indeed, taking into account this new correlation aspect for the interpretation of biological networks should allow to capture the actually activated mechanisms at the cellular level.

The development, the interest and the illustration of a method of the invention is below exposed in relation with rheumatoid arthritis (RA). Of course, the invention is not restricted to RA, it extends to any pathology with which at least one biological pathway can be associated. As a further example, said pathology may be systemic lupus erythematosus (SLE), multiple sclerosis (MS), Sjögren's syndrome, type I diabetes, dermatomyositis, etc....

The method of the invention for determining a level of activity of a biological pathway from a biological sample of a patient suffering from a pathology comprises the following steps:
measuring the level of expression of at least three genes from a group of individuals, control individuals or patients suffering from the pathology, for which the biological pathway is inactive, said at least three genes being associated to said biological pathway, for establishing a negative reference, and
measuring level of expression of said at least three genes from a group of individuals, control individuals or patients suffering from the pathology, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein
if the correlation score is lower to the predetermined threshold that is indicative of the non activity of the biological pathway, and
if the correlation score is upper or equal to the predetermined threshold that is indicative of the activity of the biological pathway.

In accordance with the present invention, the phrase "control individual" encompasses any individual wherein said pathology is not diagnosed, in particular it encompasses healthy individuals, patients suffering from any other pathology than said pathology, asymptomatic patients suffering from said pathology.

The present invention also concerns a method for *in vitro* establishing a prognosis to develop a pathology for an healthy individual, by determining a level of activity of a biological pathway, said method comprising the steps of:
providing a sample from the healthy individual,
measuring the level of expression of at least three genes from a group of individuals, control individuals or patients suffering from the pathology, for which the biological pathway is inactive, said at least three genes being associated to said biological pathway, for establishing a negative reference, and
measuring level of expression of said at least three genes from a group of individuals, control individuals or patients suffering from the pathology, for which the biological pathway is active, for establishing a positive reference, and
measuring the level of expression of said at least three genes of said healthy individual in the sample, and
comparing the level of expression of said at least three genes in the sample of said healthy individual with the level of expression of said at least three genes of the negative reference and determining a value C- which corresponds to a correlation level between the level of expression of said at least three genes of said negative reference and the level of expression of said at least three genes of said control individual, and
comparing the level of expression of said at least three genes in the sample of said healthy individual with the level of expression of said at least three genes of the positive reference and determining a value C+ which corresponds to a correlation level between the level of expression of said at least three genes of said positive reference and the level of expression of said at least three genes of said control individual, and
establishing a ratio C+/C- which gives a correlation score, wherein

if the correlation score is lower to the predetermined threshold that is indicative of the non activity of the biological pathway, that means that the healthy individual could not develop the pathology, and

if the correlation score is upper or equal to the predetermined threshold that is indicative of the activity of the biological pathway, that means that the healthy individual could develop the pathology.

In another embodiment, the invention also relates to a method for *in vitro* establishing a diagnosis of a pathology for a patient, by determining a level of activity of a biological pathway, said method comprising the steps of:
providing a sample from the patient,
measuring the level of expression of at least three genes from a group of individuals, control individuals or patients suffering from the pathology, for which the biological pathway is inactive, said at least three genes being associated to said biological pathway, for establishing a negative reference, and
measuring level of expression of said at least three genes from a group of individuals, control individuals or patients suffering from the pathology, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein

if the correlation score is lower to the predetermined threshold that is indicative of the non activity of the biological pathway, that means that the patient does not suffer from the pathology associated with the activity of the biological pathway, and

if the correlation score is upper or equal to the predetermined threshold that is indicative of the activity of the biological pathway, that means that the patient suffers from the pathology associated with the activity of the biological pathway.

As mentioned above, the invention also pertains to the use of the above-described method for targeted/individualized therapies.

Hence, the present invention also concerns a method for assessing whether a patient having a pathology is in need of a drug administration, said drug interacting directly or indirectly with a biological pathway, in which the biological activity of the biological pathway is determined and said biological activity is associated with said pathology, said method comprising the steps of:
providing a sample from the patient,
measuring the level of expression of at least three genes from a group of individuals, control individuals or patients, for which the biological pathway is inactive, said at least three genes being associated to said biological pathway, for establishing a negative reference, and
measuring level of expression of said at least three genes from a group of individuals, control individuals or patients, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein

if the correlation score is lower to the predetermined threshold that is indicative of the non activity of the biological pathway, that means that the patient needs administration of the drug , and

if the correlation score is upper or equal to the predetermined threshold that is indicative of the activity of the biological pathway, that means that the patient does not need administration of the drug.

A further subject of the invention is a method for monitoring the treatment response of a patient to the administration of a drug, said drug interacting directly or indirectly with a biological pathway, in which the biological activity of the biological pathway is determined and said biological activity is associated with a pathology, said method comprising the steps of:
providing a sample from the patient,
measuring the level of expression of at least three genes from a group of individuals, control individuals or patients, for which the biological pathway is inactive, said at least three genes being associated to said biological pathway, for establishing a negative reference, and
measuring level of expression of said at least three genes from a group of individuals, control individuals or patients, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein

if the correlation score is lower to the predetermined threshold that is indicative of the non activity of the biological pathway, that means that the patient is a bad responder to the drug, and

if the correlation score is upper or equal to the predetermined threshold that is indicative of the activity of the biological pathway, that means that the patient is a good responder to the drug.

Preferred embodiments of the methods in accordance with the invention are below disclosed, said embodiments being taken alone or in combination.

Hence, according to an preferred embodiment, the level activity of said at least three genes is determined by analyzing the expression level of nucleic acids or proteins in said sample. Said nucleic acids comprise RNAs or cDNAs obtained from said RNAs including long and small RNAs such as mRNAs, miRNAs.

A biological sample from the patient may be a tissue sample or a fluid sample, such as a sample of blood, plasma, serum, urine, synovial fluid and cerebrospinal fluid.

In their study, the inventors selected the signature of interferon (IFN)-inducible genes as an example to study correlation levels between genes composing that signature.

Accordingly, preferred methods as described above involve a biological pathway of type I interferon (type I IFN).

Indeed, the increase of IFN regulated genes has been reported in different diseases like rheumatoid arthritis (RA), systemic lupus erythematosus (SLE) (4), systemic sclerosis (8), multiple sclerosis (9) and in tissues from patients with Sjögren's syndrome (10), type I diabetes (11-13) and dermatomyositis (12). But, to characterize the IFN signature, an IFN "score" is calculated from common methods, i.e, the IFN "score" is calculated for each patient and control based on the average expression of genes which composed the signature. However, as explained above, this approach does not take into account the co-regulation of these IFN inducible genes. In fact, genes with similar functions usually are co-expressed under certain experimental conditions only.

The following experimental part illustrates, by way of example and not by way of limitation, the development of a method of the invention wherein said biological pathway concerns the expression of a human type I interferon (IFN) and the pathology is rheumatoid arthritis (RA), said method involving 35 genes associated with said pathway.

### FIGURES

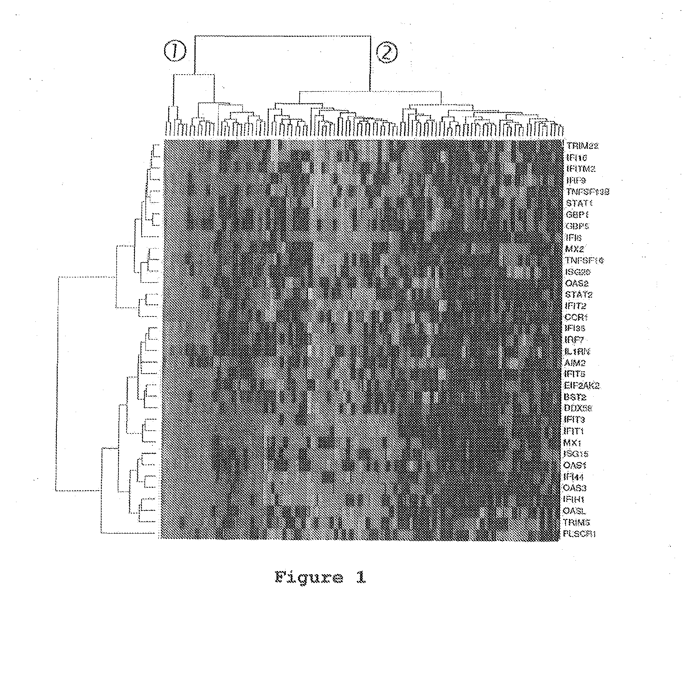

**Figure 1****. Genes expression profiles from the IFN signature.**
   Unsupervised hierarchical clustering of 35 IFN-inducible genes that distinguish rheumatoid arthritis (RA) patients IFN^{high} (dendrogram①) from RA patients IFN^{low} (dendrogram ②). Each row represents a gene; each column shows the expression for 35 IFN-inducible genes expressed by each patients. Dark grey indicates genes that are expressed at higher levels and light grey indicates genes that are expressed at lower levels.

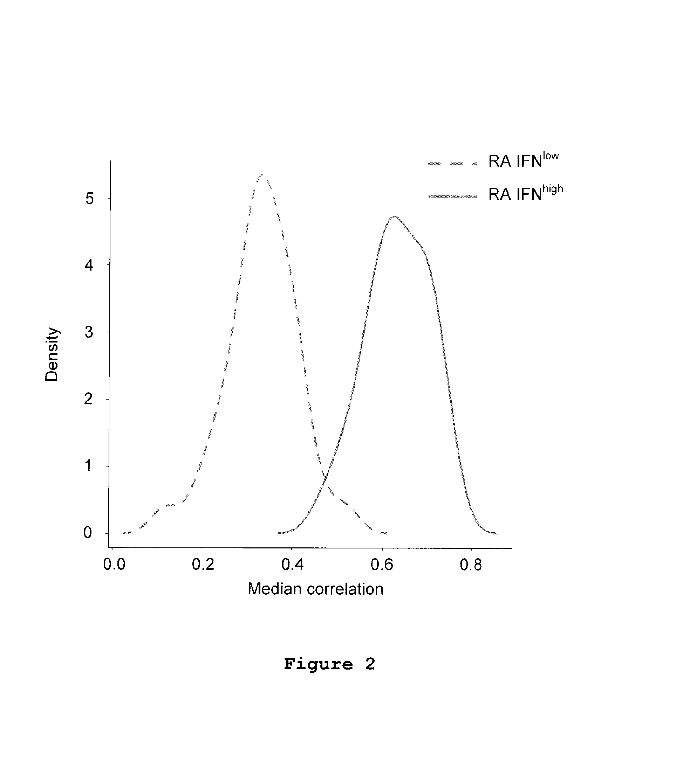

**Figure 2****. Correlation profiles from sub-groups of rheumatoid arthritis (RA) patients.**
   A correlation index was defined for each gene of the IFN signature as the median of its correlations with the remaining genes. Thus, the correlation profiles for the different groups, RA IFN^{low} (dotted line) and RA IFN^{high} (continuous line), are represented using the 35 calculated correlation indexes. The median values of the correlation indexes obtained from the different groups are 0.33 and 0.63, respectively.

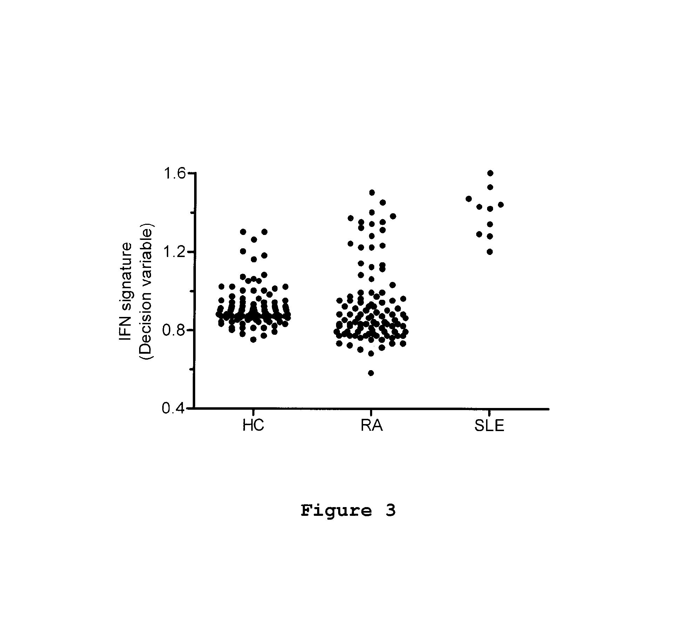

**Figure 3****. Stratification of individuals according to the IFN signature**. Each point represents a single individual with the decision variable calculated from the Classification Algorithm based on a Biological Signature (CABS). The shaded box indicates the normal range according to the rule of the CABS: If *D_{high_low} ≥ 1* "high *signature"* and If *D_{high_low}* < *1* "low signature" knowing that *D_{high_low}* = *COR_{high}* / *COR_{low}.*

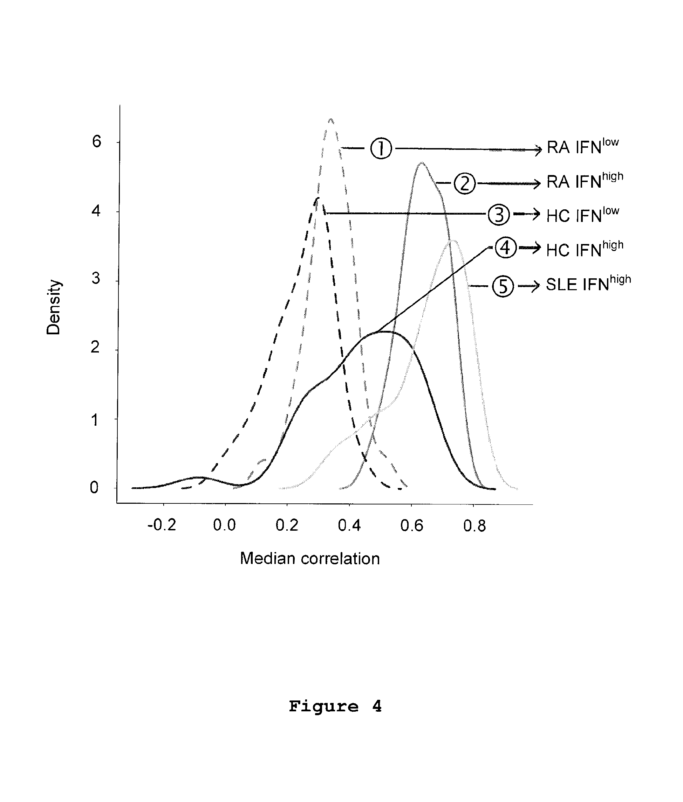

**Figure 4****. Correlation profiles from the different groups.** A correlation index was defined for each gene of the IFN signature as the median of its correlations with the remaining genes. Thus, the correlation profiles for the different groups: healthy controls (HC) IFN^{low} (③), HC IFN^{high} (④), rheumatoid arthritis patients (RA) IFN^{low} (①) and RA IFN^{high} (②) and systemic lupus erythematosus patients (SLE) IFN^{high} (⑤), are represented using the 35 calculated correlation indexes from the IFN signature genes. The median values of the correlation indexes obtained from the different groups are0.27, 0.44, 0.33, 0.63 and 0.68, respectively.

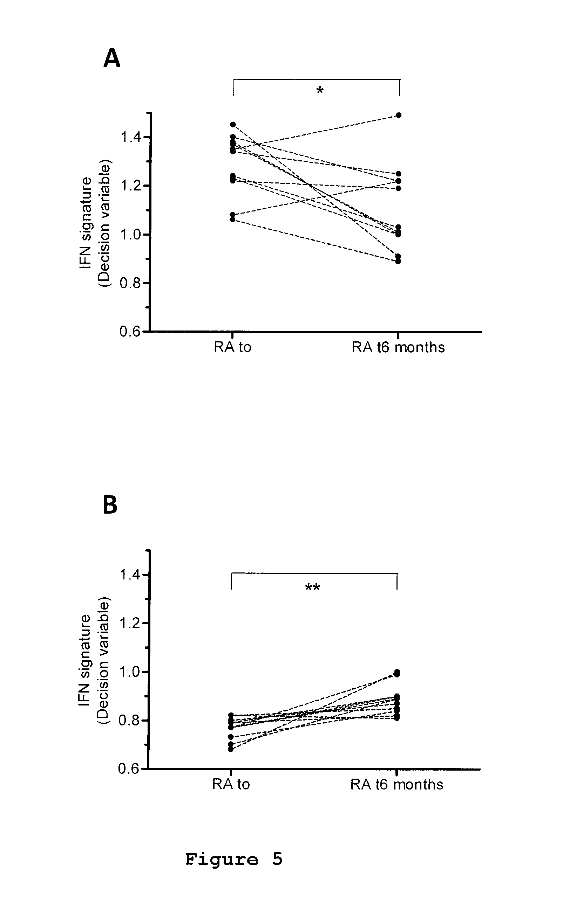

**Figure 5****. Follow-up the IFN signature in patients with rheumatoid arthritis (RA) treated with anti-TNF.**
   Each point represents a single individual with the decision variable calculated from the Classification Algorithm based on a Biological Signature (CABS). The shaded box indicates the normal range according to the rule of the CABS: If *D_{high_low} ≥* 1 "high *signature*" and If *D_{high_low} <* 1 "low signature" knowing that *D_{high_low}* = *COR_{high}* / *COR_{low}.* The Wilcoxon signed rank test was used to evaluate the statistical significance between patients before and after anti-TNF treatment A) (**p* = 0.0186) B) (***p* = 0.002). The results show that a high IFN signature is conserved after anti-TNF treatment (Figure 5A), while a significant increase was observed in RA IFN^{low} six months after treatment (Figure 5B).

### EXAMPLE ILLUSTRATING THE PREVENT INVENTION.

### METHODS

### Patients and controls

102 RA patients fulfilling the revised American College of Rheumatology 1987 criteria for RA were enrolled. Their clinical characteristics are shown in Table 1. As an IFN positive control group (IFN^{high}), 10 systemic lupus erythematosus patients (SLE) fulfilling the American College of Rheumatology criteria for the SLE were studied. In addition, 100 age- and sex-matched healthy control subjects (HC) without any familial history of RA, autoimmune disease and concomitant medication were also recruited. All subjects provided written informed consent and the study was approved by the local Ethical Committee for clinical research. The table 1 describes the demographic and clinical characteristics of the patients and healthy control subjects.

**TABLE 1: Demographic and clinical characteristics of the patients and control subjects**

| | RA (n=102) | SLE (n=10) | C (n=100) |
|---|---|---|---|
| *Demographic data* | | | |
| Age^{a} | 50(40,3-60) | 37(34-44) | 57(52-63) |
| Sex: Female, Male | 79F, 23M | 10F | 86F, 14M |
| *Disease characteristics* | | | |
| ESR^{a} | 18 (8-44) | NA | NA |
| Rheumatoid Factor pos.(%) | 70 (68,6) | NA | NA |
| Disease duration (years) ^{a} | 5 (2-9) | 4 (3-6) | NA |
| Disease activity | 4,2 (3,3-5,2)^{b} | 13 (12-17.5)^{c} | NA |
| *Medication* | | | |
| MTX (%) | 87 (85,3) | NA | NA |
| MTX dose^{a} | 15 (15-20) | NA | NA |

| | | | |
|---|---|---|---|
| Median (Q1-Q3) ^{b} DAS28: Disease Activity Score ^{c} SLEDAI: Systemic Lupus Erythematosus Disease Activity Index ESR: Erythrocyte Sedimentation Rate; MTX: Methotrexate | | | |

**Sample collection, processing and microarray hybridization** Peripheral blood samples were collected in PAXgene^{™} Blood RNA tubes (PreAnalytix, Hilden, Germany) in order to stabilize mRNA (14). Blood samples were incubated at room temperature for 2 h, and then stored at -20°C until RNA extraction according to the manufacturer's instructions. Briefly, RNA was isolated using the PAXgene^{™} Blood RNA kit (PreAnalytix). Following cell lysis, nucleic acids were pelleted and treated with a buffer containing proteinase K. After digestion with a RNase-free DNase (Qiagen, Valencia, CA, USA), RNA was subsequently purified on PAXgene^{™} spin columns and eluted in 80 µl of elution buffer. RNA integrity was assessed using RNA 6000 nano chips and the Agilent 2100 Bioanalyzer (Agilent Technologies, Waldbronn, Germany) according to the manufacturer's instructions. The RNA integrity number (RIN) was obtained from the entire electrophoretic trace of the RNA sample. cDNA was synthesized from 50 ng of total RNA using the WT-Ovation^{™} System (NuGEN, San Carlos, CA, USA) powered by Ribo-SPIA^{™} technology. Fragmented cDNA was end labeled with a biotin-conjugated nucleotide analog (DLR-1a; Affymetrix, Santa Clara, CA, USA) using terminal transferase (Roche Diagnostics, Mannheim, Germany). Fragmented and labeled cDNA was hybridized for 18 h at 50°C in a hybridization solution containing 7% DMSO. Hybridization was performed using GeneChip^{®} Human Genome U133 Plus 2.0 arrays (Affymetrix), containing 54,675 probe sets corresponding to 38,500 identified genes. After washing, chips were stained with streptavidin-phycoerythrin according to Affymetrix EukGE-WS2v4 protocol using the Fluidic FS450 station. The microarrays were read with the GeneChip^{®} Scanner 3000 (Affymetrix). Affymetrix GeneChip Operating Software version 1.4 (GCOS) was used to manage Affymetrix GeneChip array data and to automate the control of GeneChip fluidics stations and scanners.

### Data analysis

### Data processing

Expression data were generated using the Robust Multi-array Average (RMA) method (15) implemented in the Affy package of the Bioconductor microarray analysis environment (http://www.bioconductor.org). The RMA method consists of three steps: background adjustment, quantile normalization (16) and probe set summary of the log-normalized data applying a median polishing procedure. Before subsequent patient stratification, non informative genes showing very low expression levels and low variability across microarrays were excluded.

### Biclustering and functional enrichment analyses

The SAMBA algorithm (Statistical-Algorithmic Method for Bicluster Analysis) implemented in EXPANDER 4.0.3 (EXPression ANalyzer and DisplayER) was used for the biclustering (17). This algorithm uses probabilistic modeling of the data and theoretical graph techniques to identify such subsets of genes that behave similarly across a subset of patients (18).

The TANGO algorithm (Tool for Analysis of GO enrichment), implemented in EXPANDER 4.0.3, was used to identify the biological significance of these biclusters (17).

### Classification Algorithm based on a Biological Signature (CABS)

A classification algorithm was developed to identify individuals with or without the type I IFN gene biological signature. Applied to the IFN-inducible genes, the CABS is divided into three steps.

*Step 1 Prototype construction:* Two groups of RA patients (IFN^{high}; IFN^{low}) were identified from the hierarchical clustering representing the 35 IFN-inducible genes which characterized the IFN signature (Figure 1). The prototype was defined from these two groups. Median expression values was calculated in the two groups. Prototype Pi was defined from group *i*; the vector (*Gi₁*,.., *Gi_{M}*) represents the expression of the prototype *Pi*, where *i* is high or low, *Gij* is the median expression of gene *j* in group *i, M* is the size of the IFN signature.

*Step 2 Decision variable calculation:* For a given individual, the IFN gene expression profiles corresponding to a vector with a size of 35 genes were extracted. Pearson correlation of genes related to the IFN signature was evaluated with both prototypes and denoted *COR_{high}* and *COR_{low}.* The decision variable calculation was given by the ratio between these two correlations: *D_{high_low}* = *COR_{high}* / *COR_{low}*

*Step 3 Decision making:* The following rule was applied to classify the individuals. High IFN signature was assigned if *D_{high_low} ≥ 1*. Inversely, low IFN signature was attributed if *D_{high_low} < 1*.

### RESULTS

### Analysis of heterogeneity with the biclustering method

The study of biological data heterogeneity was conducted with a biclustering approach. This method using the SAMBA algorithm performs clustering on genes and conditions simultaneously in order to identify subsets of genes that show similar expression patterns across specific subsets of patients and vice versa. After data filtering, 121 biclusters were identified from 9,856 selected probe sets. To draw a clear picture of these co-expressed gene groups, the TANGO algorithm was used for GO functional enrichment analysis. The identified biclusters were represented by 15 functional biological processes (Table 2)

**TABLE 2: Ontological analysis of the 121 biclusters obtained from the 102 RA patients**

| Biological process | Corrected p-value |
|---|---|
| immune response ^{a} | 0.001 |
| response to virus ^{a} | 0.001 |
| generation of precursor metabolites and energy | 0.001 |
| oxidative phosphorylation | 0.001 |
| cellular defense response | 0.001 |
| biosynthetic process | 0.001 |
| Cytolysis | 0.001 |
| response to biotic stimulus | 0.001 |
| signal transduction | 0.001 |
| response to wounding | 0.001 |
| cell surface receptor linked signal transduction | 0.007 |
| cell communication | 0.013 |
| B cell activation | 0.037 |
| cell adhesion | 0.048 |

| | |
|---|---|
| Processes with corrected p value < 0.05 were considered significant (17). ^{a} Biological terms composed of 95% IFN mediated immunity genes. | |

To focus on the IFN signature, the "immune response" and "response to virus" ontology groups, which represent a broad composite family, were selected. Interestingly, within this subgroup, 95% of 37 genes were known to be induced by IFN. The list of these 35 genes is presented in the right column of figure 1.

### Activation of IFN pathway in a sub-group of RA patients

To visualize the expression profiles of the 35 IFN-response genes among all RA patients and to investigate their interactions, a hierarchical clustering was performed with the Spotfire Decision Site 8.2.1. This clustering separated the samples into two main groups, one of patients with RA (n = 26/102, 25.5%) with high expression (Figure 1, dendrogram ①) of this set of IFN-inducible genes (IFN^{high}) and another (n = 76/102, 74.5%) with lower expression (Figure 1, dendrogram ②) (IFN^{low}).

### Characterization of the IFN signature based on a correlation approach

The expression pattern of 35 IFN-response genes was defined as the "IFN signature". To go further in the description of the IFN-induced genes, the correlation levels between the co-expressed genes were assessed in the two groups. Interestingly, the analysis revealed disparities between correlation levels. The group associated with high IFN expression level showed a better correlation (R_{median} = 0.63) than the other one (R_{median} = 0.33), with a significant difference (p = 8.46E-13), suggesting a functional difference in the activated state of these genes (Figure 2). A classification algorithm was applied to obtain a better characterization of the IFN signature based on the correlation of the 35 gene expression levels. The results showed that the IFN signature presented a large variation between individuals (Figure 3). 15/100 HC (15%), 22/102 RA patients (22%) and 10/10 SLE patients (100%) with a decision variable ≥1 for the high signature (IFN^{high}) were identified, while the remainder of individuals, with a decision variable <1, were defined as IFN^{low}. From the sub-groups identified by the CABS, the comparison of the correlation profiles showed heterogeneous distributions (Figure 4). Similarities were observed, first between RA and SLE patients associated with a high IFN signature presenting a median correlation of 0.63 and 0.68 respectively; second between RA patients and HC IFN^{low} presenting a median correlation of 0.33 and 0.27 respectively. However, despite a similar level of correlation (R_{median} = 0.44), the shape of the distribution curve (Figure 4, ④) is very different from that seen for the high RA or SLE patients and for the low RA or controls. This could suggest a very heterogeneous activation status of genes in this group of controls.

### Effect of TNF inhibition on IFN pathway activation

The functional relationship between TNF inhibition and possible changes in IFN pathway activation was studied. CABS was used to assess the correlation levels in RA patients before and after anti-TNFα treatment. Out of the subgroup of 43 RA patients treated with anti-TNF, 22 RA patients (11 RA IFN^{high} and 11 RA IFN^{low} ; infliximab n = 6, etanercept n = 10 and adalimumab n = 6) were evaluated at 6 months for treatment response using the DAS28 criteria. Although the values appeared quite heterogeneous, a statistical significant decrease (p = 0.0186) of the correlation level was observed in patients associated with high IFN signature (Figure 5A). In contrast, a statistical significant increase (p = 0.002) of correlation levels was seen in RA patients with low IFN signature before treatment (Figure 5B). Despite a significant increase, the majority of these RA patients IFN^{low} did not reach the threshold of positivity. No statistical association was observed between the molecular stratification of RA patients (IFN^{high} / IFN^{low}) and the clinical characteristics presented in table 1 or the response to treatment at 6 months.

The method of the present invention permits to identify truly active biological networks associating only with high levels of correlation of biological signature components. This new correlation aspect for the interpretation of biological networks allows capturing the actually activated mechanisms at the cellular level.

Such correlation-based approach can be advantageously applied to investigate the dynamics of evolution of cellular mechanisms like response to treatment. As an example, in the context of RA, the inventors have applied this method to monitor patients treated by anti-TNF therapy.

Interestingly also, the method illustrating the present invention and using CABS allows to pinpoint type I IFN signaling as a means to stratify RA patients even starting with whole blood transcriptomics analysis from samples collected in PAXgene tubes. Similar analyses can be performed for the other identified biclusters, highlighting the obvious advantage of whole blood transcriptomics. Using the example of the IFN signature, the use of correlations shows interest in the characterization of the genes sharing both an expression pattern and a biological function. The use of expression correlations is a better way to obtain a global picture of an activated signature in various disease conditions.

### REFERENCES BIBLIOGRAPHIQUES

1. Eisen MB, Spellman PT, Brown PO, Botstein D. Cluster analysis and display of genome-wide expression patterns. Proc Natl Acad Sci U S A. 1998;95(25):14863-8.
2. Ben-Dor A, Chor B, Karp R, Yakhini Z. Discovering local structure in gene expression data: the order-preserving submatrix problem. J Comput Biol. 2003;10(3-4):373-84.
3. van der Pouw Kraan TC, Wijbrandts CA, van Baarsen LG, Voskuyl AE, Rustenburg F, Baggen JM, et al. Rheumatoid arthritis subtypes identified by genomic profiling of peripheral blood cells: assignment of a type I interferon signature in a subpopulation of patients. Ann Rheum Dis. 2007 Aug;66(8):1008-14.
4. Baechler EC, Batliwalla FM, Karypis G, Gaffney PM, Ortmann WA, Espe KJ, et al. Interferon-inducible gene expression signature in peripheral blood cells of patients with severe lupus. Proc Natl Acad Sci U S A. 2003;100(5):2610-5.
5. Baechler EC, Bauer JW, Slattery CA, Ortmann WA, Espe KJ, Novitzke J, et al. An interferon signature in the peripheral blood of dermatomyositis patients is associated with disease activity. Mol Med. 2007;13(1-2):59-68.
6. Bauer JW, Baechler EC, Petri M, Batliwalla FM, Crawford D, Ortmann WA, et al. Elevated serum levels of interferon-regulated chemokines are biomarkers for active human systemic lupus erythematosus. PLoS Med. 2006;3(12):e491.
7. Kirou KA, Lee C, George S, Louca K, Papagiannis IG, Peterson MG, et al. Coordinate overexpression of interferon-alpha-induced genes in systemic lupus erythematosus. Arthritis Rheum. 2004;50(12):3958-67.
8. Tan FK, Zhou X, Mayes MD, Gourh P, Guo X, Marcum C, et al. Signatures of differentially regulated interferon gene expression and vasculotrophism in the peripheral blood cells of systemic sclerosis patients. Rheumatology (Oxford). 2006;45(6):694-702.
9. van Baarsen LG, van der Pouw Kraan TC, Kragt JJ, Baggen JM, Rustenburg F, Hooper T, et al. A subtype of multiple sclerosis defined by an activated immune defense program. Genes Immun. 2006;7(6):522-31.
10. Bave U, Nordmark G, Lövgren T, Rönnelid J, Cajander S, Eloranta ML, et al. Activation of the type I interferon system in primary Sjögren's syndrome: a possible etiopathogenic mechanism. Arthritis Rheum. 2005;52(4):1185-95.
11. Reynier F, Pachot A, Paye M, Xu Q, Turrel-Davin F, Petit F, et al. Specific gene expression signature associated with development of autoimmune type-I diabetes using whole-blood microarray analysis. Genes Immun. 2010;11(3):269-78.
12. Greenberg SA, Pinkus JL, Pinkus GS, Burleson T, Sanoudou D, Tawil R, et al. Interferon-alpha/beta-mediated innate immune mechanisms in dermatomyositis. Ann Neurol. 2005;57(5):664-78.
13. Huang X, Yuang J, Goddard A, Foulis A, James RF, Lernmark A, et al. Interferon expression in the pancreases of patients with type I diabetes. Diabetes. 1995;44(6):658-64.
14. Rainen L, Oelmueller U, Jurgensen S, Wyrich R, Ballas C, Schram J, et al. Stabilization of mRNA expression in whole blood samples. Clin Chem. 2002 Nov;48(11):1883-90.
15. Irizarry RA, Hobbs B, Collin F, Beazer-Barclay YD, Antonellis KJ, Scherf U, et al. Exploration, normalization, and summaries of high density oligonucleotide array probe level data. Biostatistics. 2003 Apr;4(2):249-64.
16. Bolstad BM, Irizarry RA, Astrand M, Speed TP. A comparison of normalization methods for high density oligonucleotide array data based on variance and bias. Bioinformatics. 2003 Jan 22;19(2):185-93.
17. Shamir R, Maron-Katz A, Tanay A, Linhart C, Steinfeld I, Sharan R, et al. EXPANDER: an integrative program suite for microarray data analysis. BMC Bioinformatics. 2005;21:6:232.
18. Tanay A, Sharan R, Shamir R. Discovering statistically significant biclusters in gene expression data. Bioinformatics. 2002;18 Suppl 1:S136-44.

## Claims

**1.** A method for determining a level of activity of a biological pathway from a biological sample of a patient suffering from a pathology, said method comprising the steps of:
measuring the level of expression of at least three genes from a group of individuals, control individuals or patients suffering from the pathology, for which the biological pathway is inactive, said at least three genes being associated to said biological pathway, for establishing a negative reference, and
measuring level of expression of said at least three genes from a group of individuals, control individuals or patients suffering from the pathology, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein
if the correlation score is lower to the predetermined threshold that is indicative of the non activity of the biological pathway, and
if the correlation score is upper or equal to the predetermined threshold that is indicative of the activity of the biological pathway.

**2.** A method for *in vitro* establishing a prognosis to develop a pathology for an healthy individual, by determining a level of activity of a biological pathway, said method comprising the steps of:
providing a sample from the healthy individual,
measuring the level of expression of at least three genes from a group of individuals, control individuals or patients suffering from the pathology, for which the biological pathway is inactive, said at least three genes being associated to said biological pathway, for establishing a negative reference, and
measuring level of expression of said at least three genes from a group of individuals, control individuals or patients suffering from the pathology, for which the biological pathway is active, for establishing a positive reference, and
measuring the level of expression of said at least three genes of said healthy individual in the sample, and
comparing the level of expression of said at least three genes in the sample of said healthy individual with the level of expression of said at least three genes of the negative reference and determining a value C- which corresponds to a correlation level between the level of expression of said at least three genes of said negative reference and the level of expression of said at least three genes of said control individual, and
comparing the level of expression of said at least three genes in the sample of said healthy individual with the level of expression of said at least three genes of the positive reference and determining a value C+ which corresponds to a correlation level between the level of expression of said at least three genes of said positive reference and the level of expression of said at least three genes of said control individual, and
establishing a ratio C+/C- which gives a correlation score, wherein
if the correlation score is lower to the predetermined threshold that is indicative of the non activity of the biological pathway, that means that the healthy individual could not develop the pathology, and
if the correlation score is upper or equal to the predetermined threshold that is indicative of the activity of the biological pathway, that means that the healthy individual could develop the pathology.

**3.** A method for *in vitro* establishing a diagnosis of a pathology for a patient, by determining a level of activity of a biological pathway, said method comprising the steps of:
providing a sample from the patient,
measuring the level of expression of at least three genes from a group of individuals, control individuals or patients suffering from the pathology, for which the biological pathway is inactive, said at least three genes being associated to said biological pathway, for establishing a negative reference, and
measuring level of expression of said at least three genes from a group of individuals, control individuals or patients suffering from the pathology, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein
if the correlation score is lower to the predetermined threshold that is indicative of the non activity of the biological pathway, that means that the patient does not suffer from the pathology associated with the activity of the biological pathway, and
if the correlation score is upper or equal to the predetermined threshold that is indicative of the activity of the biological pathway, that means that the patient suffers from the pathology associated with the activity of the biological pathway.4. A method for assessing whether a patient having a pathology is in need of a drug administration, said drug interacting directly or indirectly with a biological pathway, in which the biological activity of the biological pathway is determined and said biological activity is associated with said pathology, said method comprising the steps of:
providing a sample from the patient,
measuring the level of expression of at least three genes from a group of individuals, control individuals or patients, for which the biological pathway is inactive, said at least three genes being associated to said biological pathway, for establishing a negative reference, and
measuring level of expression of said at least three genes from a group of individuals, control individuals or patients, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein
if the correlation score is lower to the predetermined threshold that is indicative of the non activity of the biological pathway, that means that the patient needs administration of the drug , and
if the correlation score is upper or equal to the predetermined threshold that is indicative of the activity of the biological pathway, that means that the patient does not need administration of the drug.

**5.** A method for monitoring the treatment response of a patient to the administration of a drug, said drug interacting directly or indirectly with a biological pathway, in which the biological activity of the biological pathway is determined and said biological activity is associated with a pathology, said method comprising the steps of:
providing a sample from the patient,
measuring the level of expression of at least three genes from a group of individuals, control individuals or patients, for which the biological pathway is inactive, said at least three genes being associated to said biological pathway, for establishing a negative reference, and
measuring level of expression of said at least three genes from a group of individuals, control individuals or patients, for which the biological pathway is active, for establishing a positive reference, and
measuring the level of expression of said at least three genes of said patient in the sample, and
comparing the level of expression of said at least three genes in the sample of said patient with the level of expression of said at least three genes of the negative reference and determining a value C- which corresponds to a correlation level between the level of expression of said at least three genes of said negative reference and the level of expression of said at least three genes of said patient, and
comparing the level of expression of said at least three genes in the sample of said patient with the level of expression of said at least three genes of the positive reference and determining a value C+ which corresponds to a correlation level between the level of expression of said at least three genes of said positive reference and the level of expression of said at least three genes of said patient, and
establishing a ratio C+/C- which gives a correlation score, wherein
if the correlation score is lower to the predetermined threshold that is indicative of the non activity of the biological pathway, that means that the patient is a bad responder to the drug, and
if the correlation score is upper or equal to the predetermined threshold that is indicative of the activity of the biological pathway, that means that the patient is a good responder to the drug.

**6.** Method of anyone of claims 1 to 5, wherein the level activity of said at least three genes is determined by analyzing the expression level of nucleic acids or proteins in said sample.

**7.** Method of anyone of claims 1 to 5, wherein said nucleic acids comprises RNAs or cDNAs obtained from said RNAs including long and small RNAs such as mRNAs, miRNAs.

**8.** Method of anyone of claims 1 to 75, wherein said biological sample is a fluid sample or a tissue sample.

**9.** Method of claim 8, wherein the biological sample is selected from the group consisting of blood, plasma, serum, urine, synovial fluid and cerebrospinal fluid.

**10.** Method according to anyone of claims 1 to 9, wherein said biological pathway is the pathway of type I interferon.

**11.** Method according to anyone of claims 1 to 10, wherein said pathology is selected from the group consisting of rheumatoid arthritis and systemic lupus erythematosus.
